# EUROPEAN PATENT APPLICATION

(11) **EP 4 763 098 A1**
(43) Date of publication of application: **24.06.2026**
(21) Application number: 24307234.5
(22) Date of filing: 20.12.2024
(51) Int. Cl.: A61B 17/02, A61B 34/20

(54) **SYSTEM AND METHOD FOR ASSISTING A SURGEON TO PERFORM SHOULDER ARTHROPLASTY**

(71) Applicant: SCAP Hologram, 38400 Saint-Martin-D'Hères (FR)
(72) Inventor: HENRY, Delphine, 38400 SAINT-MARTIN-D'HÈRES (FR); CARDON, Jean-Emmanuel, 38400 SAINT-MARTIN-D'HÈRES (FR); SCHERS, Jonathan, 38400 SAINT-MARTIN-D'HÈRES (FR)
(74) Representative: Regimbeau

(57) **Abstract**

The invention concerns a system for assisting a surgeon for performing shoulder arthroplasty in a surgical field comprising a glenoid cavity of a patient's shoulder, the system comprising:
- at least one extension pole intended to be attached to an instrument, the extension pole being configured to be non-magnetic-field-disturbing by material and/or by design;
- an EM localization system comprising
- an EM transmitter;
- an EM instrument receiver attached to the extension pole, the extension pole permitting to stay away the EM instrument receiver from the instrument to which it is attached;
- an EM bone receiver intended to be located near the glenoid cavity, preferably rigidly attached to a scapula of the patient's shoulder;
the EM transmitter, the EM instrument receiver and the EM bone receiver being configured for operating together by being arranged relative to each other within a functional area preliminary defined by a range of the EM transmitter, the extension pole being configured not to interfere with a-magnetic-field in the functional area.

## Description

### FIELD OF THE INVENTION

The invention relates to a system and method for assisting a surgeon to perform shoulder arthroplasty.

### BACKGROUND OF THE INVENTION

Surgery of a bone and in particular shoulder replacement surgery is a complicated type of surgery. Shoulder replacement surgery is becoming increasingly common because of its ability to alleviate pain and restore range of motion in many patients. Shoulder arthroplasty (anatomic and reversed) has been demonstrated to successfully recover function to shoulders affected by arthrosis and rotator cuff insufficiency.

However, long-term survival rate, range of motion, and complication rate depends on the correct positioning of the implant components. Surgeons often perform pre-operative plan defining implant placement, but once they are in the surgery they follow the plan without being guided.

There is a need for helping surgeons to prepare bone surface and position implants according to preoperative plan such as glenoid and humeral implants in a surgery.

Computer assisted surgery systems are mainly optical based technology. An infra-red camera is tracking reflecting elements attached to tracked objects. When surgical site is small, with difficult access, optical tracking is challenging as surgeon can interfere with the field of view, and tracking can be lost.

### BRIEF DESCRIPTION OF THE INVENTION

The invention aims at assisting a surgeon for performing shoulder arthroplasty.

According to a first aspect, the invention concerns a system for assisting a surgeon for performing shoulder arthroplasty in a surgical field comprising a glenoid cavity of a patient's shoulder, the system comprising:
- at least one extension pole intended to be attached to an instrument, the extension pole being configured to be non-magnetic-field-disturbing by material and/or by design;
- an EM localization system comprising
   - an EM transmitter;
   - an EM instrument receiver attached to the extension pole, the extension pole permitting to stay away the EM instrument receiver from the instrument to which it is attached;
   - an EM bone receiver intended to be located near the glenoid cavity, preferably rigidly attached to a scapula of the patient's shoulder;
the EM transmitter, the EM instrument receiver and the EM bone receiver being configured for operating together by being arranged relative to each other within a functional area preliminary defined by a range of the EM transmitter, the extension pole being configured not to interfere with α-magnetic-field in the functional area.

The system according to the first aspect comprises one or more of the following features alone in combination:
- It comprises a retractor assembly adapted for retracting tissues around the glenoid cavity, the retractor assembly comprising at least one retractor configured to be non-magnetic-field-disturbing by material and/or by design.
- The EM transmitter, the EM instrument receiver and the EM bone receiver are intended to be disposed relative to each other at a distance between 5 and 15 cm, preferably 10 cm in the functional area.
- It comprises a main support configured to be non-magnetic-field-disturbing by material and/or by design intended to be disposed in the functional area, the EM transmitter being arranged on the main support.
- A check plate is rigidly attached to the main support, the check plate comprising at least one receiving site for an instrument, the check plate allowing a calibration or a control of the calibration of the instrument relative to the EM transmitter.
- The check plate is configured to be non-magnetic-field-disturbing by material and/or by design.
- The extension pole comprises a sleeve intended to receive an instrument, the instrument comprising a distal end and a proximal end, the distal end being adapted to be in the functional area around the glenoid, the proximal end being adapted to be surrounded by the sleeve.
- The extension pole comprises a rigid arm extending from the sleeve, a platform for supporting the EM instrument receiver extending from the rigid arm, the platform allowing the EM instrument receiver to be as close as possible to the EM transmitter end without disturbing the manipulation of the instrument
- The extension pole comprises a handle extending from the sleeve, the handle allowing the instrument to be manipulated, a platform for supporting the EM instrument receiver extends from the handle, the platform allowing the EM instrument receiver to be as close as possible to the EM transmitter without disturbing the manipulation of the instrument.
- The handle comprises a weight so that the handle is weighted for balancing the instrument when the instrument is operated in the surgery field around the glenoid cavity.
- A non-magnetic-field-disturbing material is plastic or titanium.
- It comprises at least one instrument on which an extension pole is attached, the instrument can be chosen in the following group: a reamer configured for placing a pin, reaming the surface of the coracoid for preparing the surface of the glenoid cavity; a drill configured for drilling the glenoid cavity to obtain a hole for receiving a pin guidance, the pin guidance permitting a placement of a glenoid implant and screw placement; and a registration probe configured for designating a surface to be registered.

According to a second aspect, the invention concerns a computer implemented method for assisting a surgeon for performing shoulder arthroplasty in a surgical field comprising a glenoid cavity of a patient's shoulder, the surgical field comprising:
- at least one extension pole intended attached to an instrument, the extension pole being configured to be non-magnetic-field-disturbing by material and/or by design;
- an EM localization system comprising : an EM transmitter; an EM instrument receiver attached to the extension pole, the extension pole permitting to stay away the EM instrument receiver from the instrument to which it is attached; an EM bone receiver intended to be located near the glenoid cavity, preferably rigidly attached to a scapula of the patient's shoulder; the EM transmitter, the EM instrument receiver and the EM bone receiver being arranged relative to each other within a functional area preliminary defined around the glenoid cavity,
the method comprising a step of receiving information from the EM transmitter and each EM instrument receiver and a step of locating the at least one EM instrument receiver with respect to the EM bone receiver during the manipulation of the at least one instrument during the surgery.

According to one embodiment, an instrument being a registration probe, the method comprising a registration step comprising a step of acquiring points or surface(s) belonging to the glenoid cavity, the points or surface(s) being designed via the registration probe, the location of each point or surface(s) allowing registering of the glenoid cavity with a preoperative image of the glenoid cavity.

### BRIEF DESCRIPTION OF THE DRAWINGS

Further features and advantages of the invention will be apparent from the description that follows, based on the appended drawings, wherein:
- Figure 1 illustrates a surgical field in which the invention is implemented.
- Figure 2 illustrates an instrument of a first type equipped with an extension pole according to a first embodiment.
- Figure 3, figure 4 and figure 5 illustrate an instrument of a second type equipped with an extension pole according to a second embodiment.
- Figure 6 and figure 7 illustrate a main support along with a check plate with an EM transmitter.
- Figure 8 and figure 9 illustrate a main support along with a check plate with an EM transmitter and an instrument.
- Figure 10 illustrates main steps for performing shoulder arthroplasty.

On the figures, similar elements have identical references.

### DETAILED DESCRIPTION OF EMBODIMENTS OF THE INVENTION

The description relates to a system for assisting a surgeon for performing shoulder arthroplasty, to a computer implemented method for assisting a surgeon for performing shoulder arthroplasty.

The system and the methods herein described are based on the use of electromagnetic (EM) based localization.

### Presentation of a surgical field and of a system for assisting a surgeon for performing shoulder arthroplasty

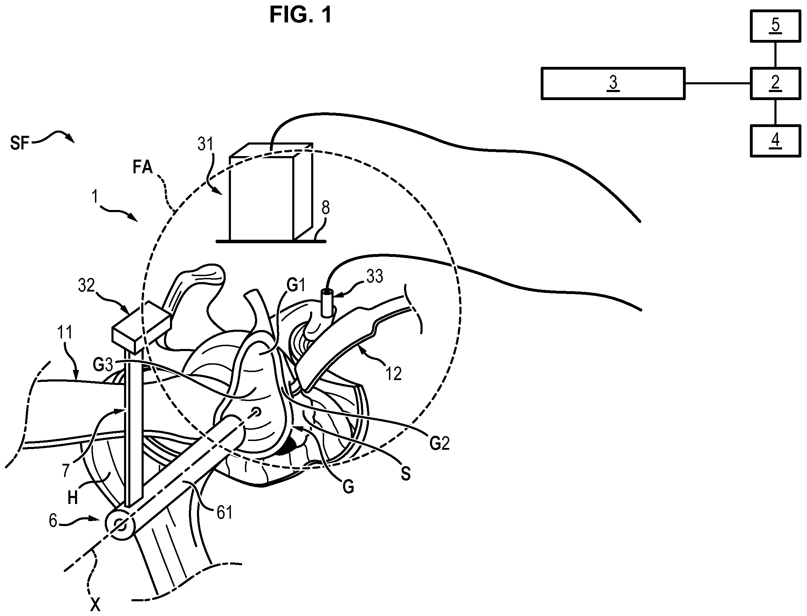

Figure 1 shows a surgical field SF in which the invention is implemented in a surgery of a shoulder in this case a shoulder arthroplasty. During the shoulder arthroplasty, a glenoidal implant (not shown) is positioned on the glenoid cavity G of the scapula S of the shoulder of the patient in front of the humerus H. The positioning of this implant requires a correct access to the glenoid cavity G. Thus, tissues around the glenoid cavity G are retracted by means of a retractor assembly 1 comprising at least two retractors 11, 12 allowing an access to the glenoid cavity G during the surgery. Around the glenoid G the surgon needs to manipulate the instruments.

The glenoid cavity G comprises a superior part G1 corresponding to the upper part of the glenoid at the level of the clavicle, an anterior part G2, and a posterior part G3 according to the patient's anatomy.

The invention aims to assist a surgeon during shoulder arthroplasty by providing a system for performing the procedure in the surgical field SF comprising a glenoid cavity G of a patient's shoulder.

This system generally comprises a processing unit 2 connected to a localization system 3. A storage unit 4 and a display unit 5 are connected to the processing unit 2 for storing data acquired and for displaying images useful for the surgery.

Several instruments 6 for the surgery are manipulated by the surgeon. For locating each instrument during the surgery, the system for assisting a surgeon comprises at least one extension pole 7 intended to be attached to an instrument 6

The localization system 3 comprises an EM transmitter 31 and at least two EM receivers 32, 33. By processing the transmitted and received signals, the processing unit 2 permits to locate each EM receiver relative to each other and thus permits to locate each instrument 6 during the surgery. The EM transmitter 31, the EM receivers are located into a functional area FA defined by the range of the EM transmitter 31.

The EM transmitter 31 is preferably supported by a main support 8 located in the functional area FA. However, the EM transmitter 31 can be supported by any structure in functional area FA.

### Localization system

The localization system 3 comprises the EM transmitter 31 located in the surgery field SF and defines the functional area FA, at least one EM instrument receiver 32 (one for each instrument) and an EM bone receiver 33.

The EM bone receiver 33 is attached to a body part. This EM bone receiver 33 is important since the other EM receivers are located relative to this EM bone receiver which is not moving during the surgery. The EM bone receiver is positioned so that it withstands vibration of the instrument and other phenomenon which occur during surgery. The EM bone receiver 32 is preferably rigidly attached to the scapula. More generally, the EM bone receiver 32 is attached to a reference structure intended to be fixed and unaltered during the surgery, at the tip of the coracoid for example.

Each EM instrument receiver 32 is rigidly attached to a corresponding instrument to be tracked or localized. The instrument 6 comprises an elongated body 61 extending along a longitudinal axis X.

The EM transmitter 31, the EM receivers 32, 33 are located into the surgical field SF into a functional area FA defined by the range of the EM transmitter 31 (the circle on figure 1). Indeed, the range of the EM transmitter 31 decreases as a function of d³, d being the distance between the EM transmitter 31 and one each of the EM receivers 32, 33. It is therefore necessary to locate the EM receivers 32, 33 relative to the EM transmitter 31 so that the electromagnetic signals are sufficient to ensure accurate localization. The EM transmitter 31 is preferably attached to the patient itself or more generally as close as possible to the glenoid G of the patient without risking disturbing the surgeon during the surgery.

The EM transmitter 31 and the EM receiver 32, 33 are arranged in the functional area FA so that
(i) each EM receivers 32, 33 are in a range of 5 to 15 centimeters, preferably 10 centimeters from the EM transmitter 31; and
(ii) magnetic-field-disturbing object(s) are avoided between the EM transmitter 31 and the EM receivers 32, 33.

The idea is to define in the surgical field SF perturbing emitting area(s), i.e., that is/are susceptible to perturb the localization system 3 based on EM waves. A perturbing area is for instance around one or more element(s) susceptible to perturb the localization system 3.

Using an EM localization system 3 is very advantageous since it increases access, precision, and navigation during procedures compared to optical localization system for instance.

### Instrument / extension pole

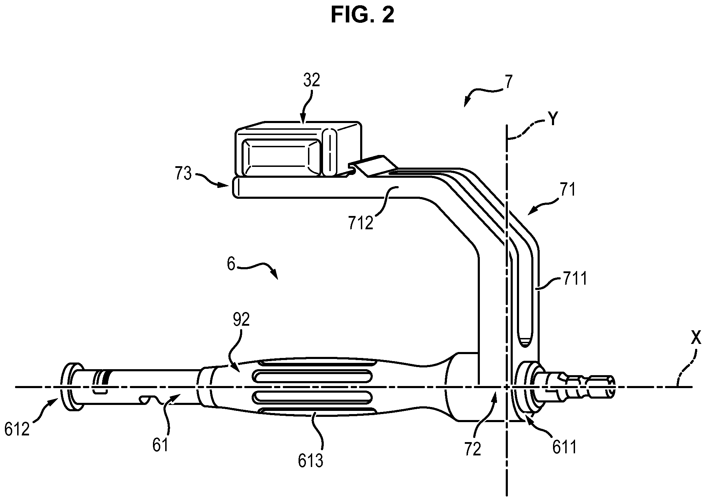

Figure 2 illustrates an instrument 6 of a first type equipped with an extension pole 7 according to a first embodiment supporting the EM instrument receiver 32. This instrument 6 is for instance a reamer. As already indicated, the instrument comprises an elongated body 61 extending along a longitudinal axis X.

The elongated body 61 comprises a proximal end 611 and a distal end 612 and a manipulation area 613 between the proximal end 611 and the distal end 612. The manipulation area 613 is the area where the surgeon grasps the instrument 6 for its manipulation. The distal end 612 is intended to be manipulated in the functional area FA and to be in contact with the glenoid G during the surgery.

The proximal end 611 is intended to be connected to a power cable (not shown) for powering the instrument 6.

The extension pole 7 is attached to the instrument 6 through its elongated body 61 and extends from the proximal end 611. In particular, the extension pole 7 comprises a sleeve 72 surrounding the elongated body 61 on its proximal end 611 and a rigid arm 71 extending from the sleeve 72. Thus, the sleeve 72 extends from the proximal end 611 along the longitudinal axis X towards the distal end 612 and permits to attach the extension pole 7 to the instrument 6 as such. This sleeve 72 can be adjustable enabling secure attachments to the instrument 6. More generally, the extension pole 7 can be attached to the instrument 6 through any fastening means.

The rigid arm 71 comprises a first part 711 and a second part 712, the sleeve 72 extending from the first part 711. The first part 711 is elongated and extends along a longitudinal axis Y perpendicularly to the longitudinal axis X. The second part 712 extends from the first part 711 in distance and parallel to the elongated body 61 in the direction of the distal end 612. A platform 73 is an extension of the second part 712. The platform 73 supports the EM instrument receiver 32 so that it is positioned above the instrument 6.

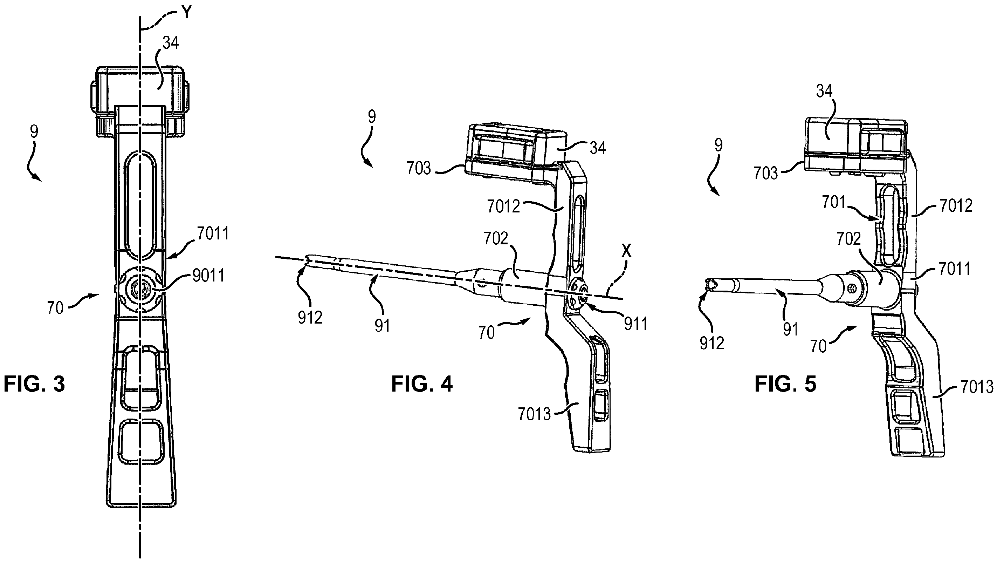

Figures 3, 4 and 5 illustrate an instrument 9 of a second type equipped with extension pole 70 according to a second embodiment supporting the EM instrument receiver 34. This instrument 9 is for instance a drill and comprises an elongated body 91 extending along a longitudinal axis X. The elongated body 91 comprises a proximal end 911 and a distal end 912. The instrument 9 of the second type differs from the instrument 6 of the first type in that it is not intended to be manipulated through the elongated body 91. The distal end 912 is intended to be manipulated in the functional area FA and to be in contact with the glenoid G during the surgery. The instrument 9 is intended to be connected to a power cable (not shown) for powering the instrument 9.

The extension pole 70 is attached to the instrument 9 through its main body 91 with a sleeve 702 surrounding the elongated body 91 on its proximal end 911 in a similar way as described for the instrument of the first type. A handle 7011 extends from the sleeve 702 and allows manipulation of the instrument. The handle 7011 is elongated and extends along a longitudinal axis Y perpendicularly to the longitudinal axis X of the elongated body 91 of the instrument 9 in two opposite directions. The handle 7011 comprises a first part 7012 and a second part 7013 extending in the opposite directions. A platform 703 is an extension of the first part 7012. The platform 703 supports the EM instrument receiver 34 so that it is positioned above instrument 9.

For balancing the instrument 9 during its manipulation the handle 74 is preferably weighed on its second part 7013. A weight can be added or the second part 7013 can be designed to be heavier than the first part 7012.

The instrument 9 according to the second embodiment differs from the instrument 6 according to the first embodiment in that it cannot be manipulated via its main body 91. The corresponding extension pole 70 for this instrument 9 is similar to the one used with the instrument 6 of the first type embodiment except that an handle is required.

When used during the surgery, each instrument 6, 9 is equipped with the corresponding extension pole 7, 70 on which a corresponding EM receiver 32, 34 is disposed: an EM reamer receiver and/or an EM drill receiver.

The extension pole 7, 70 can be detachable.

The EM receiver can be the same for each instrument and is interchangeable.

Also, the choice of the extension pole 7, 70 depends on how the instrument is handled.

The use of such an extension pole 7, 70 permits to keep the EM instrument receiver 32, 34 away from the instrument 6, 9 as such. The idea is to have the EM instrument receiver 32, 34 as close as possible from the EM transmitter 31 during the surgery without obstructing its manipulation

Indeed, the instrument comprises materials (for instance metal) that can interfere with the EM field.

The extension pole is in close contact with the EM instrument receiver, which is highly sensitive to magnetic disturbance. Extension pole 7, 70 is configured to be non-magnetic-field-disturbing (by its material and/or design). A non-magnetic-field-disturbing material is for instance plastic or titanium. A non-magnetic-field-disturbing design means that this property can be obtained with the design as such.

Advantageously, the extension pole 7, 70 is in one piece and can be manufactured by 3D printing.

The extension pole 7, 70 has a known geometry and the relative position of the platform 73, 703 the corresponding EM instrument receiver 32, 34 with the distal tip 612, 912 of the instrument 6, 9 are known ensuring accurate localization of the instrument 6, 9.

More generally, it is possible to provide several extensions poles 7, 70 of different sizes or shapes, as long as the extension pole 7, 70 supports the EM instrument receiver 32, 34 to keep it as close as possible to the EM transmitter 31 during surgery without hindering the surgeon or the patient.

### Retractor assembly

As explained above, the retractor assembly 1 is used for retracting the tissues and comprises at least a posterior retractor 11, an anterior retractor 12 and if necessary, a superior retractor (not shown) according to the patient's anatomy. In that case, the superior retractor is between the posterior retractor 11 and the anterior retractor 12.

At least one retractor 11 is in particular configured to be non-magnetic-field-disturbing by material and/or design (see above). Here again this property permits to avoid interference with the localization system 3 using EM waves. Indeed, usually the retractor assembly is made of metal bringing metallic interferences leading to poor tracking accuracy.

### Main support

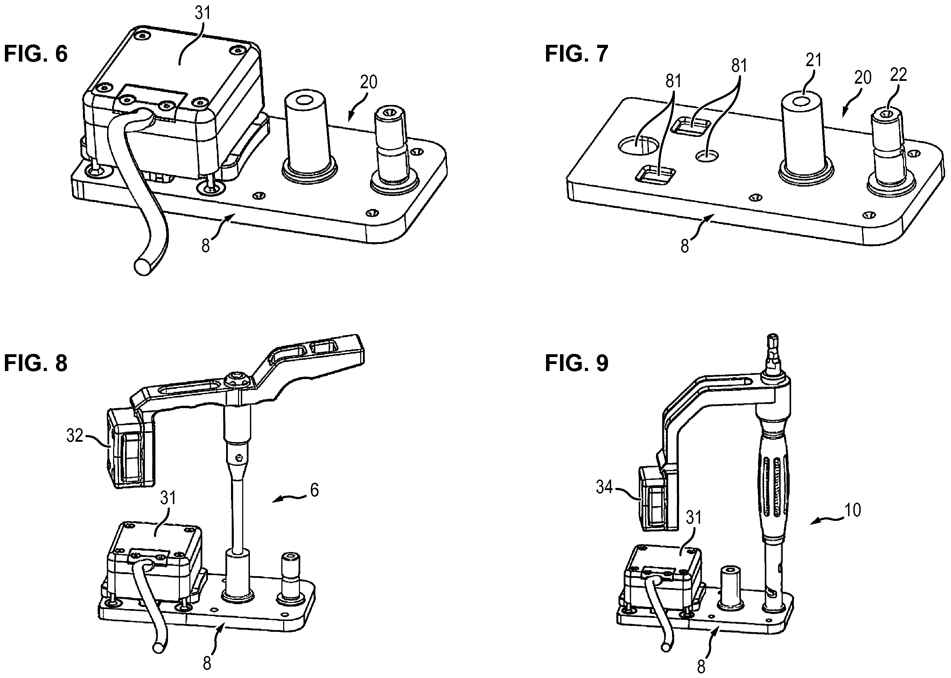

A main support 8 is located in the functional area FA and supports advantageously the EM transmitter 31. Figures 6 and 7 illustrate a main support 8 according to a preferred embodiment.

The main support 8 is preferably configured to be non-magnetic-field-disturbing by material and/or by design. This main support 8 is a plate with fixing components 81. As illustrated, the fixing components 81 are female imprints adapted for receiving complementary male imprints located on the EM transmitter 31 to be supported. The man skilled in the art will understand that any type of fixing element can be used.

In one embodiment, a check plate 20 is rigidly attached to the main support 8, forming a single piece with the main support 8. The check plate 20 includes at least one receiving site 21, 22 for an instrument 6, 10. Receiving site can be any shape complementary to the instrument, for example adjusted cylinder, divot, fins, depending on the type of desired check. It allows the EM receiver 32, 34 to be calibrated relative to the EM transmitter 31. Indeed, since the geometry of the main support 8 is known, as is the relative position of the receiving site 21, 22 with respect to the main support 8, it is possible, when positioning an instrument in a receiving site 21, 22, to check that the computed location of the instrument corresponds to the geometrically known location derived from the position of the receiving site 21, 22 with respect to the main support 8 and thus to the EM transmitter 31. If this is not the case, a calibration can be carried out in order to restore the correspondence.

This calibration can be carried out once before the surgery. Also, during the surgery, the check plate 20 enables verification of the calibration.

Again, the check plate 20 is preferably configured to be non-magnetic-field-disturbing by material and/or design. In other words, it is designed so that it does not interfere with the magnetic field.

### Computer-aided surgical navigation system for performing shoulder arthroplasty

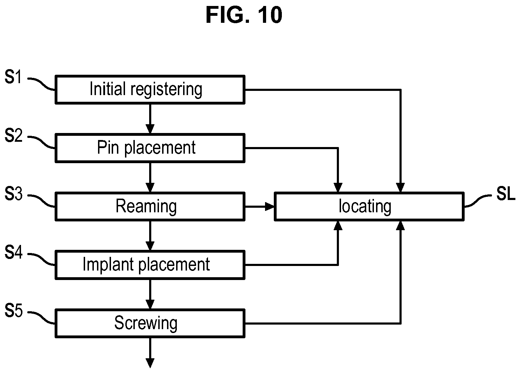

As illustrated on figure 10, the shoulder arthroplasty comprises the main following steps using at least three instruments: a drill and a reamer and a registration probe each of them having its corresponding extension pole with its EM instrument receiver:
- Step S1: Initially registering of the glenoid cavity with a preoperative image of the glenoid by means of the registration probe;
- Step S2: Pin placement comprising a drilling of the glenoid cavity and a positioning of a positioning pin by means of the drill to control for example orientation and position
- Step S3: Reaming of the glenoid cavity for preparing the surface of the glenoid cavity by means of the reamer, to control for example orientation, position and depth
- Step S4: Glenoid implant placement, with determined orientation, position, depth, peripheral screw position, distance to the bony surface for grafting if implant is not flush to the bone preparation
- Step S5: Screwing of the glenoid implant;

To this end, the surgeon is advantageously helped by the above-described system and the following instruments on which an extension pole is attached:
A probe to register glenoid anatomic structure and allow CT scan super imposition to the scapula
A drill guide configured for drilling the glenoid cavity to obtain a hole for receiving a pin guidance, the pin guidance permitting position and orientation of the reamer
A reamer configured for sliding in the pin reaming the surface of the coracoid for preparing the surface of the glenoid cavity with reaming depth control in addition to position and orientation.
The same drill can then also be used to preparer glenoid bone to receive additional fixing device, for example peripheral screw or peripheral wedges.

In particular, during each step, the localization system 3 locates (step SL) each instrument in the surgery field SF and in particular in the function area FA.

The initial registering (step S1) involves matching pre-operative data with data acquired during surgery, so that the planned procedure can be done. Preoperatively, a 3D model of the Scapula is obtained by segmenting the bone of interest in images from any type of imaging system (e.g. CT scan). During surgery, a calibrated probe with an EM receiver is used to collect points on the bone surface (in areas accessible to scanning). These points are expressed in the coordinate system of the EM bone receiver, which is rigidly attached to the bone. A mathematical optimization is used to compute a rigid transformation that maps the digitized points onto the preoperative bone surface. Once this has been computed, calibrated instruments with EM receivers can be displayed in preoperative images and on the 3D bone model.

The display unit 5 can be used for displaying the preoperative image initially registered with the function area FA for controlling the surgery that has been planned, the location of each body part and instrument can be visible on this image.

## Claims

1. A system for assisting a surgeon for performing shoulder arthroplasty in a surgical field (SF) comprising a glenoid cavity (G) of a patient's shoulder, the system comprising:
- at least one extension pole (7, 70) intended to be attached to an instrument (6, 9), the extension pole (7, 70) being configured to be non-magnetic-field-disturbing by material and/or by design;
- an EM localization system (3) comprising
- an EM transmitter (31);
- an EM instrument receiver (32, 34) attached to the extension pole (7, 70), the extension pole (7, 70) permitting to stay away the EM instrument receiver (32, 34) from the instrument to which it is attached;
- an EM bone receiver (33) intended to be located near the glenoid cavity (G), preferably rigidly attached to a scapula of the patient's shoulder;
the EM transmitter (31), the EM instrument receiver (32, 34) and the EM bone receiver (33) being configured for operating together by being arranged relative to each other within a functional area (FA) preliminary defined by a range of the EM transmitter (31), the extension pole (7, 70) being configured not to interfere with a-magnetic-field in the functional area (FA).

2. The system of claim 1, comprising a retractor assembly (1) adapted for retracting tissues around the glenoid cavity (G), the retractor assembly (1) comprising at least one retractor configured to be non-magnetic-field-disturbing by material and/or by design.

3. The system of claims 1 to 2, wherein the EM transmitter (31), the EM instrument receiver (32, 34) and the EM bone receiver (33) are intended to be disposed relative to each other at a distance between 5 and 15 cm, preferably 10 cm in the functional area (FA).

4. The system of claims 1 to 3, comprising a main support (8) configured to be non-magnetic-field-disturbing by material and/or by design intended to be disposed in the functional area (FA), the EM transmitter (31) being arranged on the main support (8).

5. The system of claim 4, wherein a check plate (20) is rigidly attached to the main support (8), the check plate (20) comprising at least one receiving site (21, 22) for an instrument, the check plate allowing a calibration or a control of the calibration of the instrument relative to the EM transmitter (31).

6. The system of claim 5, wherein the check plate (20) is configured to be non-magnetic-field-disturbing by material and/or by design.

7. The system of claims 1 to 6, wherein the extension pole (7, 70) comprises a sleeve (72, 702) intended to receive an instrument (7, 9), the instrument (7, 9) comprising a distal end (612, 912) and a proximal end (611, 911), the distal end (612, 912) being adapted to be in the functional area around the glenoid (G), the proximal end 611, 911) being adapted to be surrounded by the sleeve (72, 702).

8. The system of claim 7, wherein the extension pole (7) comprises a rigid arm (71) extending from the sleeve (72), a platform (73) for supporting the EM instrument receiver (32) extending from the rigid arm (71), the platform allowing the EM instrument receiver (32) to be as close as possible to the distal end (612) without disturbing the manipulation of the instrument (6)

9. The system of claim 7, wherein the extension pole (70) comprises a handle (7011) extending from the sleeve (702), the handle (7011) allowing the instrument (70) to be manipulated, a platform (703) for supporting the EM instrument receiver (34) extends from the handle (7011), the platform allowing the EM instrument receiver (34) to be as close as possible to the distal end (912) without disturbing the manipulation of the instrument (9).

10. The system of claim 9, wherein the handle (7011) comprises a weight so that the handle (7011) is weighted for balancing the instrument (9) when the instrument is operated in the surgery field around the glenoid cavity (G).

11. The system of claims 1 to 10, wherein a non-magnetic-field-disturbing material is plastic or titanium.

12. The system of any one of claims 1 to 11, comprising at least one instrument (6, 9) on which an extension pole (7, 70) is attached, the instrument can be chosen in the following group: a reamer configured for placing a pin, reaming the surface of the coracoid for preparing the surface of the glenoid cavity; a drill configured for drilling the glenoid cavity to obtain a hole for receiving a pin guidance, the pin guidance permitting a placement of a glenoid implant and screw placement; and a registration probe configured for designating a surface to be registered.

13. A computer implemented method for assisting a surgeon for performing shoulder arthroplasty in a surgical field (SF) comprising a glenoid cavity (G) of a patient's shoulder, the surgical field (SF) comprising:
- at least one extension pole (7, 70) intended attached to an instrument, the extension pole (7, 70) being configured to be non-magnetic-field-disturbing by material and/or by design;
- an EM localization system (3) comprising : an EM transmitter (31) r; an EM instrument receiver (32, 34) attached to the extension pole (7, 70), the extension pole (7, 70) permitting to stay away the EM instrument receiver (32, 34) from the instrument to which it is attached; an EM bone receiver (33) intended to be located near the glenoid cavity (G), preferably rigidly attached to a scapula of the patient's shoulder; the EM transmitter (31), the EM instrument receiver (32, 34) and the EM bone receiver (33) being arranged relative to each other within a functional area (FA) preliminary defined around the glenoid cavity (G),
the method comprising a step of receiving (SA) information from the EM transmitter and each EM instrument receiver and a step of locating (SL) the at least one EM instrument receiver with respect to the EM bone receiver during the manipulation of the at least one instrument during the surgery.

14. The computer implemented method as claimed in claim 13, an instrument (6, 9) being a registration probe, the method comprising a registration step (S1) comprising a step of acquiring points or surface(s) belonging to the glenoid cavity (G), the points or surface(s) being designed via the registration probe, the location of each point or surface(s) allowing registering of the glenoid cavity (G) with a preoperative image of the glenoid cavity.
